# EUROPEAN PATENT APPLICATION

(11) **EP 4 484 432 A1**
(43) Date of publication of application: **01.01.2025**
(21) Application number: 23760408.7
(22) Date of filing: 23.02.2023
(51) Int. Cl.: C07D 495/04, H10K 85/60, H10K 50/11, C09K 11/06

(54) **ORGANIC MOLECULE SERVING AS EMITTER**

(30) Priority: 23.02.2022 EP 22158235
(71) Applicant: Samsung Display Co., Ltd., Gyeonggi-do 17113 (KR)
(72) Inventor: DANZ, Michael, 76344 Eggenstein-Leopoldshafen (DE)
(74) Representative: Dr. Weitzel & Partner
(86) International application number: PCT/KR2023/002626
(87) International publication number: WO 2023/163534

(57) **Abstract**

The present disclosure relates to a pure organic molecule without a metal center, and to use of the organic molecule as an emitter in organic light-emitting diodes (OLEDs) and other optoelectronic devices.

## Description

### Technical Field

The present disclosure relates to a pure organic molecule (dye) without a metal center in Formula A, and to use of the organic molecule as an emitter in organic light-emitting diodes (OLEDs) and other optoelectronic devices.

### Background Art

Optoelectronic devices are characterized by conversion of electrical energy into photons (OLEDs, LEECs) or a reverse process of the conversion (OPV). Here, it is important that these processes proceed as efficiently as possible. Thus, in the OLED field, it is ideal to use materials having photoluminescence quantum yield as high as possible. However, as known to those skilled in the art, the fact that 25 % singlet excitons and 75 % triplet excitons are formed in OLEDs should also be considered. In this regard, typical fluorescence emitters may achieve up to 25 % internal quantum yield. However, due to the long-lived, non-luminous triplet state formed, a problem of a sharp drop in efficiency at higher currents (so-called efficiency roll-off) is caused.

To avoid this drawback and to use as many charge carriers as possible, the concept of triplet harvesting, as known to those skilled in the art, may be utilized. This usually requires the use of expensive heavy atom complexes (e.g., iridium or platinum) (see, for example, MA Baldo, DF O'Brian, ME Thompson, SR Forrest, Phys. Rev. B 1999, 60, 14422, and H Yersin, Top Curr Chem 2004, 241, 1).

Another alternative is to use materials that exhibit thermally activated delayed fluorescence (TADF). Previously formed triplet excitons may be converted to singlet excitons, and a photon may then be emitted from this state. Here, a prerequisite for such thermal repopulation is that the energetic distance between a lowest excited singlet (S₁) and triplet level (T₁) should be short. This can be achieved by, for example, using a copper-(I)-complex (in this regard, see, for example, H. Yersin, U. Monkowius, T. Fischer, T. Hofbeck, WO 2010/149748 A1, 2010), but may be also achieved by using a pure organic material (in this regard, see, for example, K. Shizu, H. Tanaka, H. Nakanotani, C. Adachi, WO 2013/172255 A1; H. Uoyama, K. Goushi, K. Shizu, H. Nomura, C. Adachi, Nature 2012, 492, 234).

Through intensive research in this field, it has been revealed that there is still a great need for new materials despite some existing concepts for these emitter materials. One reason for this need is the variety of applications (screen technology, lighting, smart packaging, etc.) and the variety of production methods (vacuum, liquid or hybrid processing). For liquid processing applications in particular, desired solubility or insolubility in special solvents should be considered so that layers may be subsequently deposited without re-etching previously deposited layers (so-called orthogonality). In addition to aforementioned material characteristics, accessibility is also relevant for commercialization. Here, not only the availability of synthetic components, but also the costs for actual synthesis of functional materials, especially purification thereof, are included.

### Disclosure

### Technical Solution

An aspect of the present disclosure relates to provision of a molecule having or being formed from a structure of Formula A. 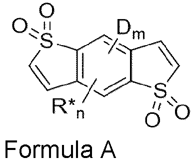
wherein, n may be 0 to 5,
m may be 1 to 6, and the sum of n and m may be 6,
Each R* may independently be
H, deuterium, F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with at least one radical R²,
one or more non-adjacent CH₂ groups may be replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR²,
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R²; an aryloxy group or heteroaryloxy group having 5 to 60 aromatic ring atoms and being substituted with one or more radicals R²; a diarylamino group, diheteroarylamino group or arylheteroarylamino group having 10 to 40 aromatic ring atoms and being substituted with one or more radicals R²; a combination of the aforementioned systems; or a cross-linkable unit QE which is cross-linked by an acid-catalyzed, base-catalyzed, thermal, or UV cross-linking process in the presence or absence of a photoinitiator or by microwave radiation,
two or more substituents among the substituents R and R' may form a mono- or polycyclic, aliphatic, aromatic and/or benzo-condensed ring system with one another,
R², identically or differently in each instance, may be
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups may be replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³,
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R³; an aryloxy group or heteroaryloxy group having 5 to 60 aromatic ring atoms and being substituted with one or more radicals R³; a diarylamino group, diheteroarylamino group or arylheteroarylamino group having 10 to 40 aromatic ring atoms and being substituted with one or more radicals R³; or a combination of the aforementioned systems,
two or more substituents R² may form a mono- or polycyclic, aliphatic, aromatic and/or benzo-condensed ring system with one another,
R³, identically or differently in each instance, may be H, deuterium, F, CF₃, or an aliphatic, aromatic and/or heteroaromatic hydrocarbon radical having 1 to 20 carbon atoms, provided that one or more H atoms may be replaced with F or CF₃, and two or more substituents R³ may form a monocyclic or polycyclic aliphatic ring system with one another, and
each D may independently be a donor group with electron-donating properties, having a structure of Formula I or II: 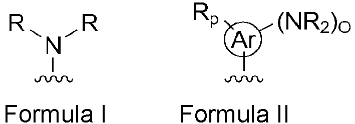
wherein
   o ≥ 1, and the sum of o and p may be (the sum of C atoms in Ar) -1,
   Ar may be an aromatic ring system having 5 to 60 aromatic ring atoms,
   the waved line indicates a position where D is bound to the aromatic backbone of the molecule of Formula A,
   each R, identically or differently in each instance, may be
   H, deuterium, F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R²,
   one or more one non-adjacent CH₂ groups may be replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR²,
   one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R²; an aryloxy group or heteroaryloxy group having 5 to 60 aromatic ring atoms and being substituted with one or more radicals R²; a diarylamino group, diheteroarylamino group or arylheteroarylamino group having 10 to 40 aromatic ring atoms and being substituted with one or more radicals R²; a combination of the aforementioned systems; or a cross-linkable unit QE which is cross-linked by an acid-catalyzed, base-catalyzed, thermal, or UV cross-linking process in the presence or absence of a photoinitiator or by microwave radiation,
   two or more substituents among the substituents R and R' may form a mono- or polycyclic, aliphatic, aromatic and/or benzo-condensed ring system with one another,
   R², identically or differently in each instance, may be
   H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
   one or more non-adjacent CH₂ groups may be replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³,
   one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R³; an aryloxy group or heteroaryloxy group having 5 to 60 aromatic ring atoms and being substituted with one or more radicals R³; a diarylamino group, diheteroarylamino group or arylheteroarylamino group having 10 to 40 aromatic ring atoms and being substituted with one or more radicals R³; or a combination of the aforementioned systems,
   two or more substituents R² may form a mono- or polycyclic, aliphatic, aromatic and/or benzo-condensed ring system with one another, and
   R³, identically or differently in each instance, may be H, deuterium, F, CF₃, or an aliphatic, aromatic and/or heteroaromatic hydrocarbon radical having 1 to 20 carbon atoms, provided that one or more H atoms may be replaced with F or CF₃, and two or more substituents R³ may form a monocyclic or polycyclic aliphatic ring system with one another.

In an embodiment, Ar may be a benzene ring, and accordingly, D may have a structure of Formula III: 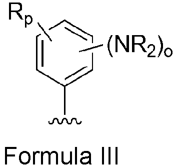 wherein
p may be 0 to 4, and o may be 1 to 5, provided that the sum of o and p may be 5, and
outside of these, the definitions set forth in connection with Formula II apply.

In one or more embodiments, D which is the donor group may have a structure of Formula IV: 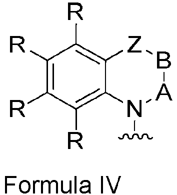 wherein
A and B may each independently be selected from the group consisting of CRR', CR, NR, and N, provided that a single bond or a double bond may be present between A and B, and a single bond or a double bond may be present between B and Z,
Z may be a direct bond or may be a divalent organic linking group including a substituted or unsubstituted C₁-C₉ alkylene group, C₂-C₈ alkenylene group, C₂-C₈ alkynylene group or arylene group, or a combination thereof, -CRR', -C=CRR', -C= NR, -NR-, -O-, -SiRR'-, -S-, -S(O)-, -S(O)₂-, an O-interrupted, substituted or unsubstituted C₁-C₉ alkylene group, C₂-C₈ alkenylene group, C₂-C₈ alkynylene group or arylene group, or a phenyl- or substituted phenyl unit,
the waved line indicates a position where D is bound to the aromatic backbone of the molecule of Formula A,
each of R and R', identically or differently in each instance, may be
H, deuterium, F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R²,
one or more non-adjacent CH₂ groups may be replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR²,
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R²; an aryloxy group or heteroaryloxy group having 5 to 60 aromatic ring atoms and being substituted with one or more radicals R²; a diarylamino group, diheteroarylamino group or arylheteroarylamino group having 10 to 40 aromatic ring atoms and being substituted with one or more radicals R²; a combination of the aforementioned systems; or a cross-linkable unit QE which is cross-linked by an acid-catalyzed, base-catalyzed, thermal, or UV cross-linking process in the presence or absence of a photoinitiator or by microwave radiation,
two or more substituents among the substituents R and R' may form a mono- or polycyclic, aliphatic, aromatic and/or benzo-condensed ring system with one another,
R², identically or differently in each instance, may be
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups may be replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³,
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R³; an aryloxy group or heteroaryloxy group having 5 to 60 aromatic ring atoms and being substituted with one or more radicals R³; a diarylamino group, diheteroarylamino group or arylheteroarylamino group having 10 to 40 aromatic ring atoms and being substituted with one or more radicals R³; or a combination of the aforementioned systems,
two or more substituents R² may form a mono- or polycyclic, aliphatic, aromatic and/or benzo-condensed ring system with one another,
R³, identically or differently in each instance, may be H, deuterium, F, CF₃, or an aliphatic, aromatic and/or heteroaromatic hydrocarbon radical having 1 to 20 carbon atoms, provided that one or more H atoms may be replaced with F or CF₃, and
two or more substituents R³ may form a mono- or polycyclic, aliphatic ring system with one another.

In an embodiment, the cross-linkable unit QE may be a compound selected from the group consisting of oxetane, alkyne, and azide for the performance of a click-reaction, as well as the following alkene derivatives:

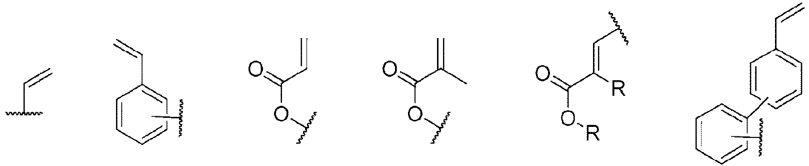

In an alternative embodiment of the present disclosure, Z may be: a covalent single bond; or a divalent organic linking group selected from a substituted and unsubstituted alkylene group (also branched or cyclic), alkenylene group, alkynylene group, arylene group, and heteroarylene group, O, NR, C=CR₂, C=NR, SiR₂ S, S(O), S(O)₂, BR, PR, and P(O)R, or a combination of the aforementioned units (e.g., an O-interrupted alkylene group (also branched or cyclic), alkenylene group, alkynylene group, arylene group, and heteroarylene group).

In an embodiment, D as a donor group with electron-donating properties may each independently be selected from: substituted and unsubstituted carbazole, substituted and unsubstituted indole, substituted and unsubstituted indoline, substituted and unsubstituted dihydroacridine, substituted and unsubstituted benzimidazole, substituted and unsubstituted 2,3,4,9-tetrahydrocarbazole, substituted and unsubstituted 1,2,3,4-tetrahydroquinoline, substituted and unsubstituted phenothiazine, substituted and unsubstituted phenothiazine, substituted and unsubstituted dihydrophenazine, and substituted and unsubstituted spiro-compounds.

The molecule according to the present disclosure provides the possibility of implementing various properties by introduction of the radicals R*, R', R², R³, and in particular R. In detail, the solubility in different solvents may be adjusted; groups (e.g., alkene, alkyne, oxetane) for cross-linking may be easily introduced; and the acceptor strength may be varied by using an electron-donating electron-accepting substituent, allowing for the emission color of the entire molecule.

An aryl group may include 6 to 40 carbon atoms. A heteroaryl group may include 2 to 40 carbon atoms and one or more heteroatoms, provided that the sum of the carbon atoms and the heteroatoms may be at least 5. The heteroatom may be particularly selected from N, O, and/or S. Here, the aryl group or the heteroaryl group may be a simple aromatic ring (i.e., benzene), a simple heteroaromatic ring (e.g., pyridine, pyrimidine, thiophene, etc.), or a condensed aryl group or heteroaryl group (e.g., naphthalene, anthracene, phenanthrene, quinoline, isoquinoline, etc.).

An aromatic ring system in the context of the present disclosure may include 6 to 60 carbon atoms. A heteroaromatic ring system in the context of the present disclosure may include 1 to 60 carbon atoms and one or more heteroatoms in the ring system, provided that the sum of the carbon atoms and the heteroatoms may be at least 5. The heteroatom may be particularly selected from N, O, and/or S. An aromatic ring system or a heteroaromatic ring system in the context of the present disclosure refers to a system which does not essentially include an aryl group or a heteroaryl group but includes several aryl groups or heteroaryl groups that can be interrupted by a nonaromatic unit (particularly less than 10 % of the atoms other than H), such as a C, N, or O atom or a carbonyl group. For example, systems such as 9,9'-spirobifluorene, 9,9-diarylfluorene, triarylamine, diarylether, stilbene, etc. shall be understood to mean an aromatic ring system in the context of the present disclosure, provided that two or more aryl groups may be, for example, substituted with a linear or cyclic alkyl group or interrupted by a silyl group. In addition, a system in which two or more aryl groups or heteroaryl groups are bonded directly to one another, such as biphenyl or terphenyl, also refers to an aromatic or heteroaromatic ring system.

A cyclic alkyl group, alkoxy group, or thioalkoxy group described herein refers to a monocyclic, bicyclic, or polycyclic group.

In the context of the present disclosure, a C₁-C₄₀ alkyl group in which individual H atoms or CH₂ groups may be also substituted with the aforementioned groups refers to, for example, radicals such as methyl, ethyl, n-propyl, i-propyl, cyclopropyl, n-butyl, i-butyl, s-butyl, t-butyl, cyclobutyl, 2-methylbutyl, n-pentyl, s-pentyl, t-pentyl, 2-pentyl, neo-pentyl, cyclopentyl, n-hexyl, s-hexyl, t-hexyl, 2-hexyl, 3-hexyl, neo-hexyl, cyclohexyl, 1-methylcyclopentyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2.2.2]octyl, 2-bicyclo[2.2.2]octyl, 2-(2,6-dimethyl)octyl, 3-(3,7-dimethyl)octyl, adamantyl, trifluoromethyl, pentafluoroethyl, 2,2,2-trifluoroethyl, 1,1-dimethyl-n-hex-1-yl-, 1,1-dimethyl-n-hept-1-yl-, 1,1-dimethyl-n-oct-1-yl-, 1,1-dimethyl-n-dec-1-yl-, 1,1-dimethyl-n-dodec-1-yl-, 1,1-dimethyl-n-tetradec-1-yl-, 1,1-dimethyl-n-hexadec-1-yl-, 1,1-dimethyl-n-octadec-1-yl-, 1,1-diethyl-n-hex-1-yl-, 1,1-diethyl-n-hept-1-yl-, 1,1-diethyl-n-oct-1-yl-, 1,1-diethyl-n-dec-1-yl-, 1,1-diethyl-n-dodec-1-yl-, 1,1-diethyl-n-tetradec-1-yl-, 1,1-diethyl-n-hexadec-1-yl-, 1,1-diethyl n-octadec-1-yl-, 1-(n-propyl)cyclohex-1-yl-, 1-(n-butyl)cyclohex-1-yl-, 1-(n-hexyl)cyclohex-1-yl-, 1-(n-octyl)-cyclohex-1-yl-, 1-(n-decyl)-cyclohex-1-yl-, etc. An alkenyl group refers to, for example, ethenyl, propenyl, butenyl, pentenyl, cyclopentenyl, hexenyl, cyclohexenyl, heptenyl, cycloheptenyl, octenyl, cyclooctenyl, or cyclooctadienyl. An alkynyl group refers to, for example, ethynyl, propynyl, butynyl, pentynyl, hexynyl, heptynyl, or octynyl. A C₁-C₄₀ alkoxy group refers to, for example, methoxy, trifluoromethoxy, ethoxy, n-propoxy, i-propoxy, n-butoxy, i-butoxy, s-butoxy, t-butoxy, or 2-methylbutoxy.

An aromatic or heteroaromatic ring system which has 5 to 60 aromatic ring atoms and may be substituted with the aforementioned radicals and linked to the aromatic or heteroaromatic system at any position refers to, for example, a group derived from benzene, naphthalene, anthracene, benzanthracene, phenanthrene, benzophenanthrene, pyrene, chrysene, perylene, fluoranthene, benzofluoranthene, naphthacene, pentacene, benzopyrene, biphenyl, biphenylene, terphenyl, terphenylene, fluorene, spiro-bifluorene, dihydrophenanthrene, dihydropyrene, tetrahydropyrene, cis- or trans-indenofluorene, cis- or trans-monobenzoindenofluorene, cis- or trans-dibenzoindenofluorene, truxene, isotruxene, spirotruxene, spiroiso-truxene, furan, benzofuran, isobenzofuran, dibenzofuran, thiophene, benzothiophene, isobenzothiophene, dibenzothiophene, pyrrole, indole, isoindole, carbazole, indolocarbazole, indenocarbazole, pyridine, quinoline, isoquinoline, acridine, phenanthridine, benzo-5,6-quinoline, benzo-6,7-quinoline, benzo-7,8-quinoline, phenothiazine, phenothiazine, pyrazole, indazole, imidazole, benzimidazole, naphthimidazole, phenanthrimidazole, pyridimidazole, pyrazineimidazole, quinoxalineimidazole, oxazole, benzooxazole, naphthoxazole, anthroxazole, phenanthroxazole, isoxazole, 1,2-thiazole, 1,3-thiazole, benzo-thiazole, pyridazine, benzopyridazine, pyrimidine, benzpyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenothiazine, phenothiazine, fluorubine, naphthyridine, aza-carbazole, benzocacarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thia-diazole, 1,2,5-thiadiazole, 1,3,4-thia-diazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine, and benzothiadiazole.

In an embodiment of the present disclosure, the organic molecule has a △E(S₁₋T₁)- value of less than 5000 cm⁻¹, less than 3000 cm-1, or less than 2000 cm⁻¹, between a lowest excited singlet (S₁) state and a triplet (T1) state therebelow.

In an embodiment of the present disclosure, the organic molecule may have a luminescence lifespan of up to 50 µs, up to 30 µs, or up to 20 µs.

In some embodiments, the organic molecule may have both the △E(S₁-T₁) value set forth herein and the luminescence lifespan set forth herein.

The organic molecule may selectively have at least one soluble group, such as at least one alkyl group. The soluble group may increase solubility of the organic molecule in a given solvent.

In an embodiment, a substituent (soluble group) to increase the solubility may be selected from the following group:
- a long-chained, branched or unbranched or cyclic alkyl chain with a length of C₁ to C₃₀,
- a long-chained, branched or unbranched or cyclic alkoxy chain with a length of C₁ to C₃₀,
- a long-chained, branched or unbranched or cyclic perfluoro alkyl chain with a length of C₁ to C₃₀, and
- a short-chained polyether with a chain length of 3 to 50.

In an embodiment of the present disclosure, the organic molecule has exactly two donor groups (D) so that the organic molecule has the structure of Formula B, C, or D: 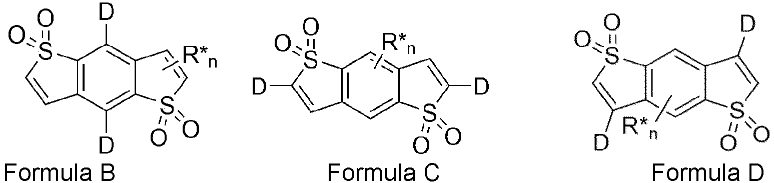 wherein
n may be 4,
each R* may independently be H, deuterium, F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R²,
one or more one non-adjacent CH₂ groups may be replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR², and
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R²; an aryloxy group or heteroaryloxy group having 5 to 60 aromatic ring atoms and being substituted with one or more radicals R²; a diarylamino group, diheteroarylamino group or arylheteroarylamino group having 10 to 40 aromatic ring atoms and being substituted with one or more radicals R²; a combination of the aforementioned systems; or a cross-linkable unit QE which is cross-linked by an acid-catalyzed, base-catalyzed, thermal, or UV cross-linking process in the presence or absence of a photoinitiator or by microwave radiation,
two or more substituents among the substituents R and R' may form a mono- or polycyclic, aliphatic, aromatic and/or benzo-condensed ring system with one another,
R², identically or differently in each instance, may be
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups may be replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³,
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R³; an aryloxy group or heteroaryloxy group having 5 to 60 aromatic ring atoms and being substituted with one or more radicals R³; a diarylamino group, diheteroarylamino group or arylheteroarylamino group having 10 to 40 aromatic ring atoms and being substituted with one or more radicals R³; or a combination of the aforementioned systems,
two or more substituents R² may form a mono- or polycyclic, aliphatic, aromatic and/or benzo-condensed ring system with one another,
R³, identically or differently in each instance, may be H, deuterium, F, CF₃, or an aliphatic, aromatic and/or heteroaromatic hydrocarbon radical having 1 to 20 carbon atoms, provided that one or more H atoms may be replaced with F or CF₃, and two or more substituents R³ may form a monocyclic or polycyclic aliphatic ring system with one another, and
each D may independently be a donor group with electron-donating properties, having a structure of Formula I or II: 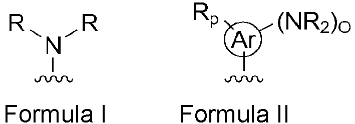
wherein
   o ≥ 1, and the sum of o and p may be (the sum of C atoms in Ar) -1,
   Ar may be an aromatic ring system having 5 to 60 aromatic ring atoms,
   the waved line indicates a position where D is bound to the aromatic backbone of the molecule of Formula A,
   each R, identically or differently in each instance, may be
   H, deuterium, F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R²,
   one or more non-adjacent CH₂ groups may be replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR²,
   one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R²; an aryloxy group or heteroaryloxy group having 5 to 60 aromatic ring atoms and being substituted with one or more radicals R²; a diarylamino group, diheteroarylamino group or arylheteroarylamino group having 10 to 40 aromatic ring atoms and being substituted with one or more radicals R²; a combination of the aforementioned systems; or a cross-linkable unit QE which is cross-linked by an acid-catalyzed, base-catalyzed, thermal, or UV cross-linking process in the presence or absence of a photoinitiator or by microwave radiation,
   two or more substituents among the substituents R and R' may form a mono- or polycyclic, aliphatic, aromatic and/or benzo-condensed ring system with one another,
   R², identically or differently in each instance, may be
   H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
   one or more non-adjacent CH₂ groups may be replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³, and
   one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R³; an aryloxy group or heteroaryloxy group having 5 to 60 aromatic ring atoms and being substituted with one or more radicals R³; a diarylamino group, diheteroarylamino group or arylheteroarylamino group having 10 to 40 aromatic ring atoms and being substituted with one or more radicals R³; or a combination of the aforementioned systems,
   two or more substituents R² may form a mono- or polycyclic, aliphatic, aromatic and/or benzo-condensed ring system with one another, and
   R³, identically or differently in each instance, may be H, deuterium, F, CF₃, or an aliphatic, aromatic and/or heteroaromatic hydrocarbon radical having 1 to 20 carbon atoms, provided that one or more H atoms may be replaced with F or CF₃; and two or more substituents R³ may form a monocyclic or polycyclic aliphatic ring system with one another.

According to an embodiment of the present disclosure, Ar may be a benzene ring, and accordingly, D may have a structure of Formula III: 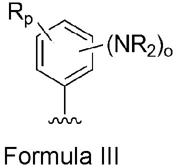 wherein
p may be 0 to 4, and o may be 1 to 5, provided that the sum of o and p may be 5, and
outside of these, the definitions set forth in connection with Formula II apply.

In some embodiments of the present disclosure, the donor group D may have a structure of Formula IV: 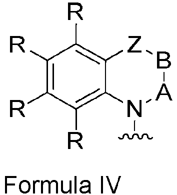 wherein
A and B may each independently be selected from the group consisting of CRR', CR, NR, and N, provided that a single bond or a double bond may be present between A and B, and a single bond or a double bond may be present between B and Z,
Z may be a direct bond or may be a divalent organic linking group including a substituted or unsubstituted C₁-C₉ alkylene group, C₂-C₈ alkenylene group, C₂-C₈ alkynylene group or arylene group, or a combination thereof, -CRR', -C=CRR', -C= NR, -NR-, -O-, -SiRR'-, -S-, -S(O)-, -S(O)₂-, an O-interrupted, substituted or unsubstituted C₁-C₉ alkylene group, C₂-C₈ alkenylene group, C₂-C₈ alkynylene group or arylene group, or a phenyl- or substituted phenyl unit,
the waved line indicates a position where D is bound to the aromatic backbone of the molecule of Formula A,
each of R and R', identically or differently in each instance, may be
H, deuterium, F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R²,
one or more non-adjacent CH₂ groups may be replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR², and
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R²; an aryloxy group or heteroaryloxy group having 5 to 60 aromatic ring atoms and being substituted with one or more radicals R²; a diarylamino group, diheteroarylamino group or arylheteroarylamino group having 10 to 40 aromatic ring atoms and being substituted with one or more radicals R²; a combination of the aforementioned systems; or a cross-linkable unit QE which is cross-linked by an acid-catalyzed, base-catalyzed, thermal, or UV cross-linking process in the presence or absence of a photoinitiator or by microwave radiation,
two or more substituents among the substituents R and R' may form a mono- or polycyclic, aliphatic, aromatic and/or benzo-condensed ring system with one another,
R², identically or differently in each instance, may be
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups may be replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³,
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R³; an aryloxy group or heteroaryloxy group having 5 to 60 aromatic ring atoms and being substituted with one or more radicals R³; a diarylamino group, diheteroarylamino group or arylheteroarylamino group having 10 to 40 aromatic ring atoms and being substituted with one or more radicals R³; or a combination of the aforementioned systems,
two or more substituents R² may form a mono- or polycyclic, aliphatic, aromatic and/or benzo-condensed ring system with one another, and
R³, identically or differently in each instance, may be H, deuterium, F, CF₃, or an aliphatic, aromatic and/or heteroaromatic hydrocarbon radical having 1 to 20 carbon atoms, provided that one or more H atoms may be replaced with F or CF₃, and two or more substituents R³ may form a monocyclic or polycyclic aliphatic ring system with one another.

In an embodiment, the cross-linkable unit QE may be a compound selected from the group consisting of oxetane, alkyne, and azide for the performance of a click-reaction, as well as the following alkene derivatives:

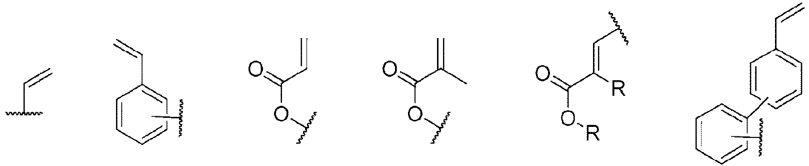

In an alternative embodiment of the present disclosure, Z may be: a covalent bond; or a divalent organic linking group selected from a substituted and unsubstituted alkylene group (also branched or cyclic), alkenylene group, alkynylene group, arylene group, and heteroarylene group, O, NR, C=CR₂, C=NR, SiR₂ S, S(O), S(O)₂, BR, PR, and P(O)R, or a combination of the aforementioned units (e.g., an O-interrupted alkylene group (also branched or cyclic), alkenylene group, alkynylene group, arylene group, and heteroarylene group).

In an embodiment, D as a donor group with electron-donating properties may each independently be selected from: substituted and unsubstituted carbazole, substituted and unsubstituted indole, substituted and unsubstituted indoline, substituted and unsubstituted dihydroacridine, substituted and unsubstituted benzimidazole, substituted and unsubstituted 2,3,4,9-tetrahydrocarbazole, substituted and unsubstituted 1,2,3,4-tetrahydroquinoline, substituted and unsubstituted phenothiazine, substituted and unsubstituted phenothiazine, substituted and unsubstituted dihydrophenazine, and substituted and unsubstituted spiro-compounds.

In some embodiments of the present disclosure, the organic molecule may have a structure of Formula E: 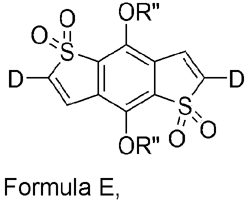 wherein
R" may be H, deuterium, CF₃, COOR², CO(NR²)₂, Si(R²)₃, B(OR²)₂, C(=O)R², P(=O)(R²)₂, S(=O)R², S(=O)₂R², O_{S}O₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy or thioalkoxy group having 3 to 40 carbon atoms and being substituted with one or more radicals R²,
one or more non-adjacent CH₂ groups may be replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR², and
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R²; an aryloxy group or heteroaryloxy group having 5 to 60 aromatic ring atoms and being substituted with one or more radicals R²; a diarylamino group, diheteroarylamino group or arylheteroarylamino group having 10 to 40 aromatic ring atoms and being substituted with one or more radicals R²; a combination of the aforementioned systems; or a cross-linkable unit QE which is cross-linked by an acid-catalyzed, base-catalyzed, thermal, or UV cross-linking process in the presence or absence of a photoinitiator or by microwave radiation,
two or more substituents among the substituents R and R' may form a mono- or polycyclic, aliphatic, aromatic and/or benzo-condensed ring system with one another,
R², identically or differently in each instance, may be
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups may be replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³, and
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R³; an aryloxy group or heteroaryloxy group having 5 to 60 aromatic ring atoms and being substituted with one or more radicals R³; a diarylamino group, diheteroarylamino group or arylheteroarylamino group having 10 to 40 aromatic ring atoms and being substituted with one or more radicals R³; or a combination of the aforementioned systems,
two or more substituents R² may form a mono- or polycyclic, aliphatic, aromatic and/or benzo-condensed ring system with one another,
R³, identically or differently in each instance, may be H, deuterium, F, CF₃, or an aliphatic, aromatic and/or heteroaromatic hydrocarbon radical having 1 to 20 carbon atoms, provided that one or more H atoms may be replaced with F or CF₃, and two or more substituents R³ may form a monocyclic or polycyclic aliphatic ring system with one another, and
each D may independently be a donor group with electron-donating properties, having a structure of Formula I or II: 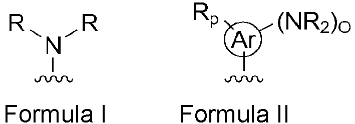
wherein
   o ≥ 1, and the sum of o and p (the sum of C atoms in Ar) may be -1;
   Ar may be an aromatic ring system having 5 to 60 aromatic ring atoms;
   the waved line indicates a position where D is bound to the aromatic backbone of the molecule of Formula A;
   each R, identically or differently in each instance, may be
   H, deuterium, F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R²,
   one or more non-adjacent CH₂ groups may be replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR²,
   one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R²; an aryloxy group or heteroaryloxy group having 5 to 60 aromatic ring atoms and being substituted with one or more radicals R²; a diarylamino group, diheteroarylamino group or arylheteroarylamino group having 10 to 40 aromatic ring atoms and being substituted with one or more radicals R²; a combination of the aforementioned systems; or a cross-linkable unit QE which is cross-linked by an acid-catalyzed, base-catalyzed, thermal, or UV cross-linking process in the presence or absence of a photoinitiator or by microwave radiation;
   two or more substituents among the substituents R and R' form a mono- or polycyclic, aliphatic, aromatic and/or benzo-condensed ring system with one another;
   R², identically or differently in each instance, may be
   H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
   one or more non-adjacent CH₂ groups may be replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³,
   one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R³; an aryloxy group or heteroaryloxy group having 5 to 60 aromatic ring atoms and being substituted with one or more radicals R³; a diarylamino group, diheteroarylamino group or arylheteroarylamino group having 10 to 40 aromatic ring atoms and being substituted with one or more radicals R³; or a combination of the aforementioned systems;
   two or more substituents R² may form a mono- or polycyclic, aliphatic, aromatic and/or benzo-condensed ring system with one another, and
   R³, identically or differently in each instance, may be H, deuterium, F, CF₃, or an aliphatic, aromatic and/or heteroaromatic hydrocarbon radical having 1 to 20 carbon atoms, provided that one or more H atoms may be replaced with F or CF₃, and two or more substituents R³ may form a monocyclic or polycyclic aliphatic ring system with one another.

In an embodiment, Ar may be a benzene ring, and accordingly, D may have a structure of Formula III: 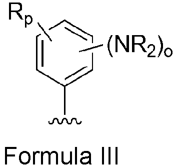 wherein
p may be 0 to 4, and o may be 1 to 5, provided that the sum of o and p may be 5, and
outside of these, the definitions set forth in connection with Formula II apply.

In some embodiments, the donor group D may have a structure of Formula IV: 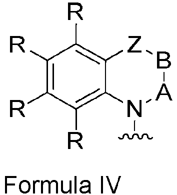 wherein
A and B may each independently be selected from the group consisting of CRR', CR, NR, and N, provided that a single bond or a double bond may be present between A and B, and a single bond or a double bond may be present between B and Z;
Z may be a direct bond or may be a divalent organic linking group including a substituted or unsubstituted C₁-C₉ alkylene group, C₂-C₈ alkenylene group, C₂-C₈ alkynylene group or arylene group, or a combination thereof, -CRR', -C=CRR', -C= NR, -NR-, -O-, -SiRR'-, -S-, -S(O)-, -S(O)₂-, an O-interrupted, substituted or unsubstituted C₁-C₉ alkylene group, C₂-C₈ alkenylene group, C₂-C₈ alkynylene group or arylene group, or a phenyl- or substituted phenyl unit;
the waved line indicates a position where D is bound to the aromatic backbone of the molecule of Formula E;
each of R and R', identically or differently in each instance, may be
H, deuterium, F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R²,
one or more non-adjacent CH₂ groups may be replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR², and
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R²; an aryloxy group or heteroaryloxy group having 5 to 60 aromatic ring atoms and being substituted with one or more radicals R²; a diarylamino group, diheteroarylamino group or arylheteroarylamino group having 10 to 40 aromatic ring atoms and being substituted with one or more radicals R²; a combination of the aforementioned systems; or a cross-linkable unit QE which is cross-linked by an acid-catalyzed, base-catalyzed, thermal, or UV cross-linking process in the presence or absence of a photoinitiator or by microwave radiation,
two or more substituents among the substituents R and R' may form a mono- or polycyclic, aliphatic, aromatic and/or benzo-condensed ring system with one another;
R², identically or differently in each instance, may be
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups may be replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³,
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R³; an aryloxy group or heteroaryloxy group having 5 to 60 aromatic ring atoms and being substituted with one or more radicals R³; a diarylamino group, diheteroarylamino group or arylheteroarylamino group having 10 to 40 aromatic ring atoms and being substituted with one or more radicals R³; or a combination of the aforementioned systems;
two or more substituents R² may form a mono- or polycyclic, aliphatic, aromatic and/or benzo-condensed ring system with one another; and
R³, identically or differently in each instance, may be H, deuterium, F, CF₃, or an aliphatic, aromatic and/or heteroaromatic hydrocarbon radical having 1 to 20 carbon atoms, provided that one or more H atoms may be replaced with F or CF₃, and two or more substituents R³ may form a monocyclic or polycyclic aliphatic ring system with one another.

In an embodiment, the cross-linkable unit QE may be a compound selected from the group consisting of oxetane, alkyne, in particular for the performance of a click-reaction, as well as the following alkene derivatives:

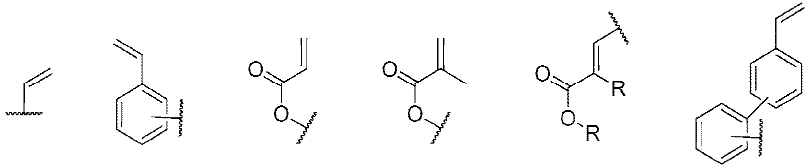

In an alternative embodiment of the present disclosure, Z may be: a covalent single bond; or a divalent organic linking group selected from a substituted and unsubstituted alkylene group (also branched or cyclic), alkenylene group, alkynylene group, arylene group, and heteroarylene group, O, NR, C=CR₂, C=NR, SiR₂ S, S(O), S(O)₂, BR, PR, and P(O)R, or a combination of the aforementioned units (e.g., an O-interrupted alkylene group (also branched or cyclic), alkenylene group, alkynylene group, arylene group, and heteroarylene group).

In an embodiment, D as a donor group with electron-donating properties may each independently be selected from: substituted and unsubstituted carbazole, substituted and unsubstituted indole, substituted and unsubstituted indoline, substituted and unsubstituted dihydroacridine, substituted and unsubstituted benzimidazole, substituted and unsubstituted 2,3,4,9-tetrahydrocarbazole, substituted and unsubstituted 1,2,3,4-tetrahydroquinoline, substituted and unsubstituted phenothiazine, substituted and unsubstituted phenothiazine, substituted and unsubstituted dihydrophenazine, and substituted and unsubstituted spiro-compounds.

In some embodiments of the present disclosure, the organic molecule may have a structure of Formula F: 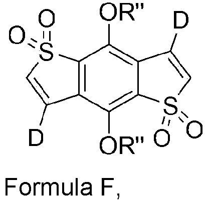 wherein
R" may be H, deuterium, CF₃, COOR², CO(NR²)₂, Si(R²)₃, B(OR²)₂, C(=O)R², P(=O)(R²)₂, S(=O)R², S(=O)₂R², O_{S}O₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy or thioalkoxy group having 3 to 40 carbon atoms and being substituted with one or more radicals R²,
one or more non-adjacent CH₂ groups may be replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR², and
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R²; an aryloxy group or heteroaryloxy group having 5 to 60 aromatic ring atoms and being substituted with one or more radicals R²; a diarylamino group, diheteroarylamino group or arylheteroarylamino group having 10 to 40 aromatic ring atoms and being substituted with one or more radicals R²; a combination of the aforementioned systems; or a cross-linkable unit QE which is cross-linked by an acid-catalyzed, base-catalyzed, thermal, or UV cross-linking process in the presence or absence of a photoinitiator or by microwave radiation;
two or more substituents among the substituents R and R' may form a mono- or polycyclic, aliphatic, aromatic and/or benzo-condensed ring system with one another,
R², identically or differently in each instance, may be
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups may be replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³,
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R³; an aryloxy group or heteroaryloxy group having 5 to 60 aromatic ring atoms and being substituted with one or more radicals R³; a diarylamino group, diheteroarylamino group or arylheteroarylamino group having 10 to 40 aromatic ring atoms and being substituted with one or more radicals R³; or a combination of the aforementioned systems;
   two or more substituents R² may form a mono- or polycyclic, aliphatic, aromatic and/or benzo-condensed ring system with one another;
R³, identically or differently in each instance, may be H, deuterium, F, CF₃, or an aliphatic, aromatic and/or heteroaromatic hydrocarbon radical having 1 to 20 carbon atoms, provided that one or more H atoms may be replaced with F or CF₃, and two or more substituents R³ may form a monocyclic or polycyclic aliphatic ring system with one another; and
each D may independently be a donor group with electron-donating properties, having a structure of Formula I or II: 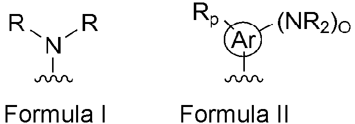
wherein
   o ≥ 1, and the sum of o and p may be (the sum of C atoms in Ar) -1;
   Ar may be an aromatic ring system having 5 to 60 aromatic ring atoms;
   the waved line indicates a position where D is bound to the aromatic backbone of the molecule of Formula A;
   each R, identically or differently in each instance, may be
   H, deuterium, F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R²,
   one or more non-adjacent CH₂ groups may be replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR², and
   one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R²; an aryloxy group or heteroaryloxy group having 5 to 60 aromatic ring atoms and being substituted with one or more radicals R²; a diarylamino group, diheteroarylamino group or arylheteroarylamino group having 10 to 40 aromatic ring atoms and being substituted with one or more radicals R²; a combination of the aforementioned systems; or a cross-linkable unit QE which is cross-linked by an acid-catalyzed, base-catalyzed, thermal, or UV cross-linking process in the presence or absence of a photoinitiator or by microwave radiation;
   two or more substituents among the substituents R and R' may form a mono- or polycyclic, aliphatic, aromatic and/or benzo-condensed ring system with one another;
   R², identically or differently in each instance, may be
   H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
   one or more non-adjacent CH₂ groups may be replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³,
   one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R³; an aryloxy group or heteroaryloxy group having 5 to 60 aromatic ring atoms and being substituted with one or more radicals R³; a diarylamino group, diheteroarylamino group or arylheteroarylamino group having 10 to 40 aromatic ring atoms and being substituted with one or more radicals R³; or a combination of the aforementioned systems;
   two or more substituents R² may form a mono- or polycyclic, aliphatic, aromatic and/or benzo-condensed ring system with one another; and
   R³, identically or differently in each instance, may be H, deuterium, F, CF₃, or an aliphatic, aromatic and/or heteroaromatic hydrocarbon radical having 1 to 20 carbon atoms, provided that one or more H atoms may be replaced with F or CF₃, and two or more substituents R³ may form a monocyclic or polycyclic aliphatic ring system with one another.

In an embodiment, Ar may be a benzene ring, and accordingly, D may have a structure of Formula III: 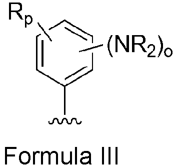 wherein
p may be 0 to 4, and o may be 1 to 5, provided that the sum of o and p may be 5, and
outside of these, the definitions set forth in connection with Formula II apply.

In one or more embodiments, the donor group D may have a structure of Formula IV: 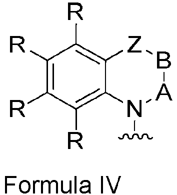 wherein
A and B may each independently be selected from the group consisting of CRR', CR, NR, and N, provided that a single bond or a double bond may be present between A and B, and a single bond or a double bond may be present between B and Z;
Z may be a direct bond or may be a divalent organic linking group including a substituted or unsubstituted C₁-C₉ alkylene group, C₂-C₈ alkenylene group, C₂-C₈ alkynylene group or arylene group, or a combination thereof, -CRR', -C=CRR', -C= NR, -NR-, -O-, -SiRR'-, -S-, -S(O)-, -S(O)₂-, an O-interrupted, substituted or unsubstituted C₁-C₉ alkylene group, C₂-C₈ alkenylene group, C₂-C₈ alkynylene group or arylene group, or a phenyl- or substituted phenyl unit;
the waved line indicates a position where D is bound to the aromatic backbone of the molecule of Formula F;
each of R and R', identically or differently in each instance, may be
H, deuterium, F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R²,
one or more non-adjacent CH₂ groups may be replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR²,
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R²; an aryloxy group or heteroaryloxy group having 5 to 60 aromatic ring atoms and being substituted with one or more radicals R²; a diarylamino group, diheteroarylamino group or arylheteroarylamino group having 10 to 40 aromatic ring atoms and being substituted with one or more radicals R²; a combination of the aforementioned systems; or a cross-linkable unit QE which is cross-linked by an acid-catalyzed, base-catalyzed, thermal, or UV cross-linking process in the presence or absence of a photoinitiator or by microwave radiation;
two or more substituents among the substituents R and R' form a mono- or polycyclic, aliphatic, aromatic and/or benzo-condensed ring system with one another;
R², identically or differently in each instance, may be
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups may be replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³,
one or more H atoms may be replaced with: deuterium, F, Cl, Br, I, CN, CF₃, or NO₂; an aromatic or heteroaromatic ring system having 5 to 60 aromatic ring atoms and being substituted with one or more radicals R³; an aryloxy group or heteroaryloxy group having 5 to 60 aromatic ring atoms and being substituted with one or more radicals R³; a diarylamino group, diheteroarylamino group or arylheteroarylamino group having 10 to 40 aromatic ring atoms and being substituted with one or more radicals R³; or a combination of the aforementioned systems,
two or more substituents R² may form a mono- or polycyclic, aliphatic, aromatic and/or benzo-condensed ring system with one another; and
R³, identically or differently in each instance, may be H, deuterium, F, CF₃, or an aliphatic, aromatic and/or heteroaromatic hydrocarbon radical having 1 to 20 carbon atoms, provided that one or more H atoms may be replaced with F or CF₃, and two or more substituents R³ may form a monocyclic or polycyclic aliphatic ring system with one another.

In an embodiment, the cross-linkable unit QE may be a compound selected from the group consisting of oxetane, alkyne, in particular for the performance of a click-reaction, as well as the following alkene derivatives:

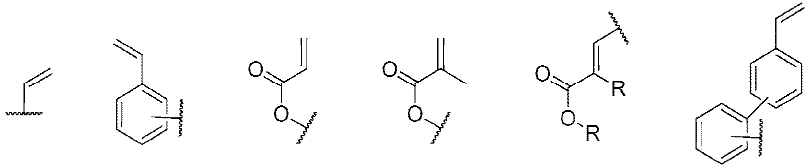

In an alternative embodiment of the present disclosure, Z may be: a covalent single bond; or a divalent organic linking group selected from a substituted and unsubstituted alkylene group (also branched or cyclic), alkenylene group, alkynylene group, arylene group, and heteroarylene group, O, NR, C=CR₂, C=NR, SiR₂ S, S(O), S(O)₂, BR, PR, and P(O)R, or a combination of the aforementioned units (e.g., an O-interrupted alkylene group (also branched or cyclic), alkenylene group, alkynylene group, arylene group, and heteroarylene group).

In an embodiment, D as a donor group with electron-donating properties may each independently be selected from: substituted and unsubstituted carbazole, substituted and unsubstituted indole, substituted and unsubstituted indoline, substituted and unsubstituted dihydroacridine, substituted and unsubstituted benzimidazole, substituted and unsubstituted 2,3,4,9-tetrahydrocarbazole, substituted and unsubstituted 1,2,3,4-tetrahydroquinoline, substituted and unsubstituted phenothiazine, substituted and unsubstituted phenothiazine, substituted and unsubstituted dihydrophenazine, and substituted and unsubstituted spiro-compounds.

In some embodiments of the present disclosure, the donor group D may have a structure of Formula V: 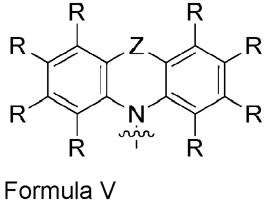 wherein
Z may be a direct bond or may be a divalent organic linking group including a substituted or unsubstituted C₁-C₉ alkylene group, C₂-C₈ alkenylene group, C₂-C₈ alkynylene group or arylene group, or a combination thereof, -CRR', -C=CRR', -C= NR, -NR-, -O-, -SiRR'-, -S-, -S(O)-, -S(O)₂-, an O-interrupted, substituted or unsubstituted C₁-C₉ alkylene group, C₂-C₈ alkenylene group, C₂-C₈ alkynylene group or arylene group, or a phenyl- or substituted phenyl unit;
the waved line indicates a position where D is bound to the aromatic backbone of the molecule of Formula A;
each of R and R', identically or differently in each instance, may be
H, deuterium, F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R²,
one or more non-adjacent CH₂ groups may be replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR²,
one or more H atoms may be replaced with: deuterium, F, Cl, Br, I, CN, CF₃, or NO₂; an aromatic or heteroaromatic ring system, each substituted with one or more radicals R² and having 5 to 60 aromatic ring atoms; an aryloxy group or heteroaryloxy group, each substituted with one or more radicals R² and having 5 to 60 aromatic ring atoms; a diarylamino group, diheteroarylamino group or arylheteroarylamino group, each substituted with one or more radicals R² and having 10 to 40 aromatic ring atoms; a combination of the aforementioned systems; or a cross-linkable unit QE which is cross-linked by an acid-catalyzed, base-catalyzed, thermal, or UV cross-linking process in the presence or absence of a photoinitiator or by microwave radiation;
two or more substituents among the substituents R and R' form a mono- or polycyclic, aliphatic, aromatic and/or benzo-condensed ring system with one another;
R², identically or differently in each instance, may be
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups may be replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³, and
one or more H atoms may be replaced with: deuterium, F, Cl, Br, I, CN, CF₃, or NO₂; an aromatic or heteroaromatic ring system, each substituted with one or more radicals R³ and having 5 to 60 aromatic ring atoms; an aryloxy group or heteroaryloxy group, each substituted with one or more radicals R³ and having 5 to 60 aromatic ring atoms; a diarylamino group, diheteroarylamino group or arylheteroarylamino group, each substituted with one or more radicals R³ and having 10 to 40 aromatic ring atoms; or a combination of the aforementioned systems;
two or more substituents R² may form a mono- or polycyclic, aliphatic, aromatic and/or benzo-condensed ring system with one another; and
R³, identically or differently in each instance, may be H, deuterium, F, CF₃, or an aliphatic, aromatic and/or heteroaromatic hydrocarbon radical having 1 to 20 carbon atoms, wherein one or more H atoms may be replaced with F or CF₃, and two or more substituents R³ form a monocyclic or polycyclic aliphatic ring system with one another.

In an embodiment, the cross-linkable unit QE may be a compound selected from the group consisting of oxetane, alkyne, and azide for the performance of a click-reaction, as well as the following alkene derivatives:

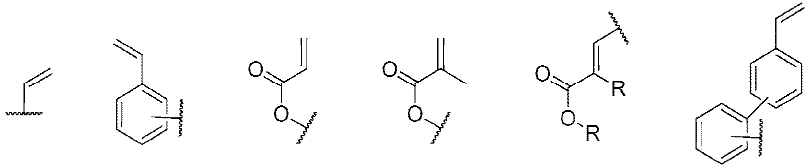

In an alternative embodiment of the present disclosure, Z may be: a covalent single bond; or a divalent organic linking group selected from a substituted and unsubstituted alkylene group (also branched or cyclic), alkenylene group, alkynylene group, arylene group, and heteroarylene group, O, NR, C=CR₂, C=NR, SiR₂ S, S(O), S(O)₂, BR, PR, and P(O)R, or a combination of the aforementioned units (e.g., an O-interrupted alkylene group (also branched or cyclic), alkenylene group, alkynylene group, arylene group, and heteroarylene group).

In some embodiments of the present disclosure, the donor group D may have a structure of Formula VI: 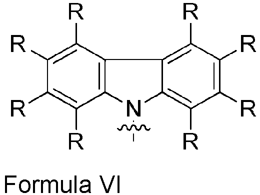 wherein
the waved line indicates a position where D is bound to the aromatic backbone of the molecule of Formula A;
each R, identically or differently in each instance, may be
H, deuterium, F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R²,
one or more non-adjacent CH₂ groups may be replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR²,
one or more H atoms may be replaced with: deuterium, F, Cl, Br, I, CN, CF₃, or NO₂; an aromatic or heteroaromatic ring system, each substituted with one or more radicals R² and having 5 to 60 aromatic ring atoms; an aryloxy group or heteroaryloxy group, each substituted with one or more radicals R² and having 5 to 60 aromatic ring atoms; a diarylamino group, diheteroarylamino group or arylheteroarylamino group, each substituted with one or more radicals R² and having 10 to 40 aromatic ring atoms; a combination of the aforementioned systems; or a cross-linkable unit QE which is cross-linked by an acid-catalyzed, base-catalyzed, thermal, or UV cross-linking process in the presence or absence of a photoinitiator or by microwave radiation;
two or more substituents among the substituents R and R' may form a mono- or polycyclic, aliphatic, aromatic and/or benzo-condensed ring system with one another,
R², identically or differently in each instance, may be
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups may be replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³,
one or more H atoms may be replaced with: deuterium, F, Cl, Br, I, CN, CF₃, or NO₂; an aromatic or heteroaromatic ring system, each substituted with one or more radicals R³ and having 5 to 60 aromatic ring atoms; an aryloxy group or heteroaryloxy group, each substituted with one or more radicals R³ and having 5 to 60 aromatic ring atoms; a diarylamino group, diheteroarylamino group or arylheteroarylamino group, each substituted with one or more radicals R³ and having 10 to 40 aromatic ring atoms; or a combination of the aforementioned systems;
two or more substituents R² may form a mono- or polycyclic, aliphatic, aromatic and/or benzo-condensed ring system with one another; and
R³, identically or differently in each instance, may be H, deuterium, F, CF₃, or an aliphatic, aromatic and/or heteroaromatic hydrocarbon radical having 1 to 20 carbon atoms, wherein one or more H atoms may be replaced with F or CF₃, and two or more substituents R³ form a monocyclic or polycyclic aliphatic ring system with one another.

In an embodiment, the cross-linkable unit QE may be a compound selected from the group consisting of oxetane, alkyne, and azide for the performance of a click-reaction, as well as the following alkene derivatives:

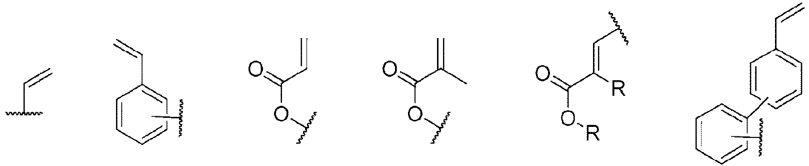

Examples of the donor group in the context of the present disclosure:

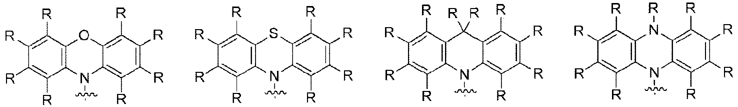

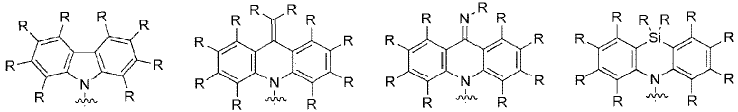

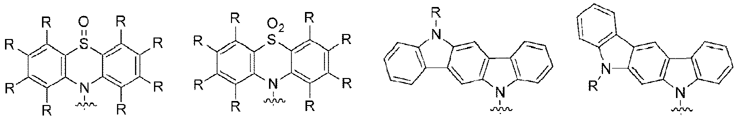

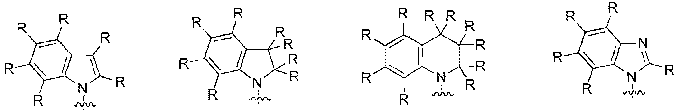

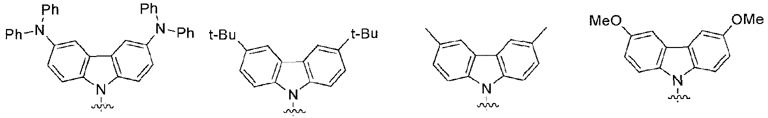

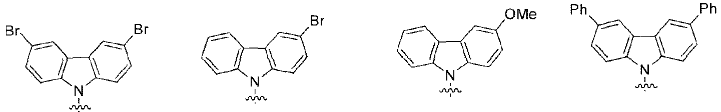

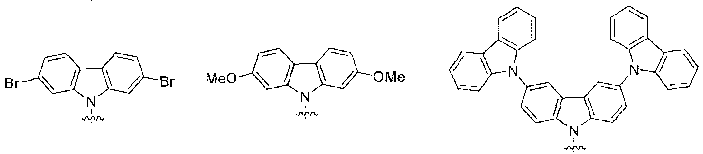

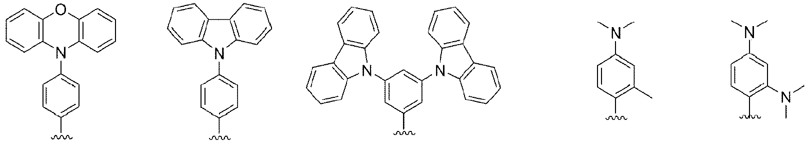

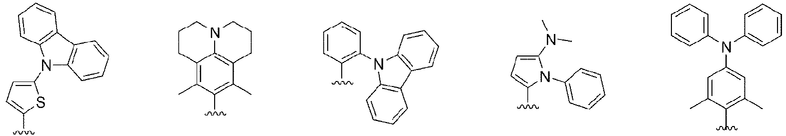

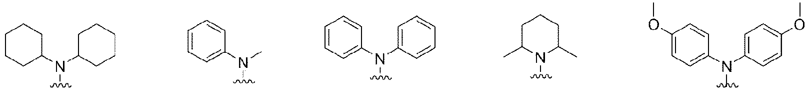

In some embodiments, the molecule in the context of the present disclosure may be substituted with 1, 2, 3, 4, 5, or 6 donor group D.

The present disclosure relates to a method of preparing the organic molecule according to the present disclosure.

In an embodiment, the method may include performing Cu-catalyzed coupling or Pd-catalyzed coupling of the corresponding bromine-substituted compound with the corresponding secondary amine. 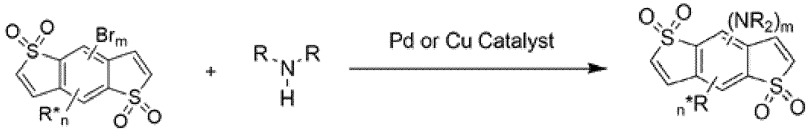 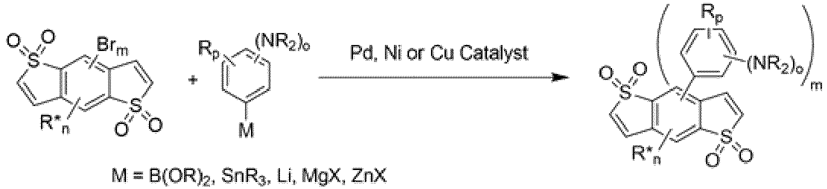 wherein the definitions set forth in Formulae A, I, and II apply, and X may be Cl, Br, or I.

In some embodiments, the method in the context of the present disclosure may further include performing substitution to increase solubility with at least one substituted selected from the following group:
- a long-chained, branched or unbranched or cyclic alkyl chain with a length of C₁ to C₃₀,
- a long-chained, branched or unbranched or cyclic alkoxy chain with a length of C₁ to C₃₀,
- a branched or unbranched or cyclic perfluoro alkyl chain with a length of C₁ to C₃₀, and
- a short-chained polyether with a chain length of 3 to 50.

In some embodiments, the method in the context of the present disclosure may further include performing substitution of the donor group D, the radical R", and/or the radical R*, with a cross-linkable unit which is cross-linked by an acid-catalyzed, base-catalyzed, thermal, or UV cross-linking process in the presence or absence of a photoinitiator or by microwave radiation:
- oxetane, azide, alkyne, and alkene derivatives for click reactions:

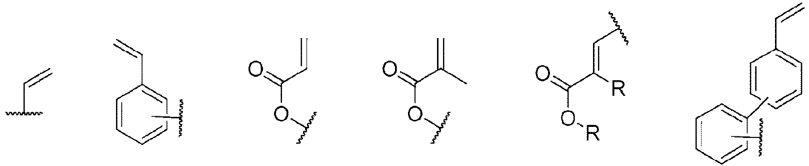

The present disclosure also relates to use of the molecule disclosed herein as an emitter or an absorber produced by a vacuum evaporation process or a solution, in an optoelectronic device, wherein the optoelectronic device may be particularly selected from the following group:
- an organic light-emitting device (OLED),
- a light-emitting electrochemical battery,
- an OLED sensor, particularly a gas sensor and an evaporation sensor that are not completely blocked from the outside world
- an organic solar battery,
- an organic field-effect transistor,
- organic laser, and
- a down-conversion element.

In an embodiment, the proportion of organic molecules in the emitter or absorber may be 1 % to 99 %. Particularly, in an OLED, especially as an emitter in an OLED, the proportion of organic molecules may be 5 % to 80 %. In some embodiments, the proportion of organic molecules in the emitter or absorber may be 100%.

The present disclosure also relates to an optoelectronic device including the organic molecule according to the present disclosure, wherein the optoelectronic device may include, particularly, an organic light-emitting device, an organic diode, an organic solar battery, an organic transistor, an organic light-emitting diode, a light-emitting electrochemical battery, an organic field-effect transistor, and an organic laser.

In an embodiment of the optoelectronic device according to the present disclosure, the compound according to the present disclosure may be used as luminescent material in an emission layer, or as a pure layer, may be used in combination with a matrix material.

In some embodiments, as a luminescent material in an emission layer of an optical light-emitting device, particularly an OLED, the proportion of the organic molecules according to the present disclosure may be 5 % to 80 %. In an embodiment of the optoelectronic device according to the present disclosure, an emission layer may be deposited on a substrate, in particularly, an anode and a cathode are deposited on a substrate, and the emission layer may be deposited between the anode and the cathode.

The emission layer may include solely the organic molecule according to the present disclosure in a concentration of 100 %, the anode and the cathode may be deposited on the substrate, and the emission layer may be deposited between the anode and the cathode.

In an embodiment, the emission layer may include, in addition to the organic molecule according to the present disclosure, a host material of which triplet (T1) and singlet (S1) energy levels are higher than triplet (T1) and singlet (S1) energy levels of the organic molecule.

In an embodiment of the optoelectronic device according to the present disclosure, a hole injection layer and an electron injection layer may be deposited between the anode and the cathode, a hole transport layer and an electron transport layer may be deposited between the hole injection layer and the electron injection layer, and the emission layer may be deposited between the hole transport layer and the electron transport layer.

In some embodiments of the present disclosure, the optoelectronic device may include a substrate, an anode, a cathode, at least one of a hole injection layer and an electron injection layer, at least one of a hole transport layer and an electron transport layer, at least one emission layer, an organic molecule of the present disclosure, and a host material of which a triplet (T1) energy level and a singlet (S1) energy level are energetically higher than a triplet (T1) energy level and a singlet (S1) energy level of the organic molecule, wherein the cathode and the anode may be deposited on the substrate, the hole injection layer and the electron injection layer may be deposited between the cathode and the anode, the hole transport layer and the electron transport layer may be deposited between the hole injection layer and the electron injection layer, and the emission layer may be deposited between the hole transport layer and the electron transport layer.

In addition, according to the present disclosure, a luminescent material is disclosed, and the luminescent material may include the organic molecule according to the present disclosure and a host material, wherein a triplet (T1) energy level and a singlet (S1) energy level of the host material may be higher than a triplet (T1) energy level and a singlet (S1) energy level of the organic molecule, the luminescent material may emit fluorescence or thermally activated delayed fluorescence, and a deltaE (S1-T1) value between a lowest excited singlet (S1) and a triplet (T1) therebelow may be less than 3000 cm-1.

The present disclosure relates to a method of preparing an optoelectronic device by using the organic molecule according to the present disclosure.

In an embodiment of the method in the context of the present disclosure, the organic molecule in the context of the present disclosure may be deposited on a carrier, and such deposition may be carried out in particular wet chemically, by colloidal suspension, or by sublimation.

In the method of preparing the optoelectronic device in the context of the present disclosure, at least one layer of the optoelectronic device may be
- coated by a sublimation process,
- coated by an organic vapor phase deposition (OVPD) process,
- coated with carrier gas sublimation, and/or
- prepared by using a solution or by a printing process.

The present disclosure relates to a method of changing emission and/or absorption properties of an electronic element, characterized by introduction of the organic molecule according to the present disclosure into a matrix material for conducting electrons or holes in the optoelectronic device.

In addition, the present disclosure relates to use of an organic molecule, in particular an organic molecule in the optoelectronic device, for converting (downconverting) UV radiation or blue light into visible light, in particular green, yellow, or red light.

### Example

Typical synthesis route: 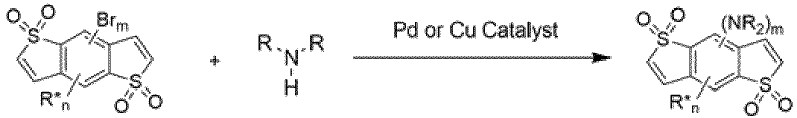 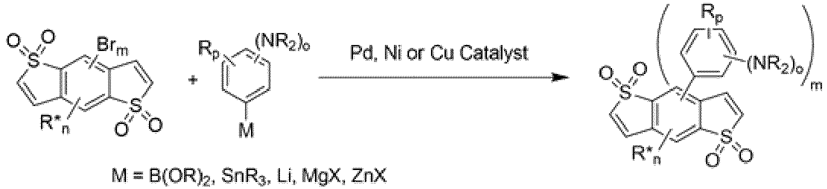 wherein the definitions in Formulae A, I, and II are applied the same, and X may be Cl, Br, or I.

In the case of direct attachment of amine, such synthesis may occur via a palladium- or copper-catalyzed reaction or a mediated reaction of single or multiple brominated disulfones with secondary amines. In a case where the donor group is attached via an aryl group, such synthesis may occur via a palladium-, nickel-, or copper-catalyzed reaction or a mediated reaction of single or multiple brominated disulfones with secondary amines.

## Claims

1. An organic molecule having a structure of Formula A: 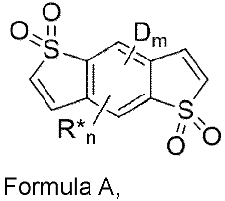 wherein
n is 0 to 5,
m is 1 to 6, and the sum of n and m is 6;
each R* is independently
H, deuterium, F, Cl, Br, I, N(R²)₂, CN, CF₃, NO₂, OH, COOH, COOR²,
CO(NR²)₂, Si(R²)₃, B(OR²)₂, C(=O)R², P(=O)(R²)₂, S(=O)R², S(=O)₂R²,
OSO₂R², a linear alkyl group, alkoxy group or thioalkoxy group having 1
to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R²,
one or more non-adjacent CH₂ groups are replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR², and
one or more H atoms are replaced with: deuterium, F, Cl, Br, I, CN, CF₃, or NO₂; an aromatic or heteroaromatic ring system, each replaced with one or more radicals R² and having 5 to 60 aromatic ring atoms; an aryloxy group or heteroaryloxy group, each substituted with one or more radicals R² and having 5 to 60 aromatic ring atoms; a diarylamino group, diheteroarylamino group or arylheteroarylamino group, each substituted with one or more radicals R² and having 10 to 40 aromatic ring atoms; a combination of the aforementioned systems; or a cross-linkable unit QE which is cross-linked by an acid-catalyzed, base-catalyzed, thermal, or
UV cross-linking process in the presence or absence of a photoinitiator or by microwave radiation,
two or more substituents among the substituents R and R' form a mono-or polycyclic, aliphatic, aromatic and/or benzo-condensed ring system with one another,
R², identically or differently in each instance, is
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups are replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³, and
one or more H atoms are replaced with: deuterium, F, Cl, Br, I, CN, CF₃, or NO₂; an aromatic or heteroaromatic ring system, each substituted with one or more radicals R³ and having 5 to 60 aromatic ring atoms; an aryloxy group or heteroaryloxy group, each substituted with one or more radicals R³ and having 5 to 60 aromatic ring atoms; a diarylamino group, diheteroarylamino group or arylheteroarylamino group, each substituted with one or more radicals R³ and having 10 to 40 aromatic ring atoms; or a combination of the aforementioned systems,
two or more substituents R² form a mono- or polycyclic, aliphatic, aromatic and/or benzo-condensed ring system with one another,
R³, identically or differently in each instance, is H, deuterium, F, CF₃, or an aliphatic, aromatic and/or heteroaromatic hydrocarbon radical having 1 to 20 carbon atoms, wherein one or more H atoms are replaced with F or CF₃, and two or more substituents R³ form a monocyclic or polycyclic aliphatic ring system with one another, and
each D is independently a donor group with electron-donating properties, having a structure of Formula I or II: 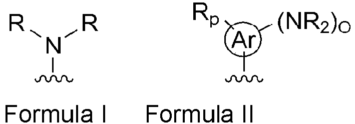
wherein
o ≥ 1, and the sum of o and p is (the sum of C atoms in Ar) -1;
Ar is an aromatic ring system having 5 to 60 aromatic ring atoms;
the waved line indicates a position where D is bound to the aromatic backbone of the molecule of Formula A;
each R, identically or differently in each instance, is
H, deuterium, F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R²,
one or more non-adjacent CH₂ groups are replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR², and
one or more H atoms are replaced with: deuterium, F, Cl, Br, I, CN, CF₃, or NO₂; an aromatic or heteroaromatic ring system, each substituted with one or more radicals R² and having 5 to 60 aromatic ring atoms; an aryloxy group or heteroaryloxy group, each substituted with one or more radicals R² and having 5 to 60 aromatic ring atoms; a diarylamino group, diheteroarylamino group or arylheteroarylamino group, each substituted with one or more radicals R² and having 10 to 40 aromatic ring atoms; a combination of the aforementioned systems; or a cross-linkable unit QE which is cross-linked by an acid-catalyzed, base-catalyzed, thermal, or UV cross-linking process in the presence or absence of a photoinitiator or by microwave radiation,
two or more substituents among the substituents R and R' form a mono- or polycyclic, aliphatic, aromatic and/or benzo-condensed ring system with one another,
R², identically or differently in each instance, is
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups are replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³, and
one or more H atoms are replaced with: deuterium, F, Cl, Br, I, CN, CF₃, or NO₂; an aromatic or heteroaromatic ring system, each substituted with one or more radicals R³ and having 5 to 60 aromatic ring atoms; an aryloxy group or heteroaryloxy group, each substituted with one or more radicals R³ and having 5 to 60 aromatic ring atoms; a diarylamino group, diheteroarylamino group or arylheteroarylamino group, each substituted with one or more radicals R³ and having 10 to 40 aromatic ring atoms; or a combination of the aforementioned systems,
two or more substituents R² form a mono- or polycyclic, aliphatic, aromatic and/or benzo-condensed ring system with one another, and
R³, identically or differently in each instance, is H, deuterium, F, CF₃, or an aliphatic, aromatic and/or heteroaromatic hydrocarbon radical having 1 to 20 carbon atoms, wherein one or more H atoms are replaced with F or CF₃, and two or more substituents R³ form a monocyclic or polycyclic aliphatic ring system with one another.

2. The organic molecule of claim 1, wherein D has a structure of Formula III: 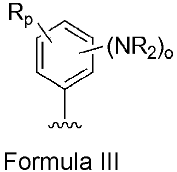 wherein
p is 0 to 4, and o is 1 to 5, provided that the sum of o and p is 5, and
outside of these, the definitions set forth in claim 1 apply.

3. The organic molecule of claim 1, wherein D has a structure of Formula IV: 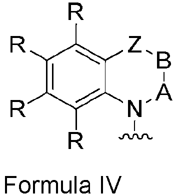 wherein
A and B are each independently selected from the group consisting of CRR', CR, NR, and N, provided that a single bond or a double bond is present between A and B, and a single bond or a double bond is present between B and Z;
Z is a direct bond or is a divalent organic linking group including a substituted or unsubstituted C₁-C₉ alkylene group, C₂-C₈ alkenylene group, C₂-C₈ alkynylene group or arylene group, or a combination thereof, -CRR', -C=CRR', -C= NR, -NR-, -O-, -SiRR'-, -S-, -S(O)-, -S(O)₂-, an O-interrupted, substituted or unsubstituted C₁-C₉ alkylene group, C₂-C₈ alkenylene group, C₂-C₈ alkynylene group or arylene group, or a phenyl- or substituted phenyl unit;
the waved line indicates a position where D is bound to the aromatic backbone of the molecule of Formula A;
each of R and R', identically or differently in each instance, is H, deuterium, azide (N₃⁻), F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R²,
one or more non-adjacent CH₂ groups are replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR², and
one or more H atoms are replaced with: deuterium, F, Cl, Br, I, CN, CF₃, or NO₂; an aromatic or heteroaromatic ring system, each substituted with one or more radicals R² and having 5 to 60 aromatic ring atoms; an aryloxy group or heteroaryloxy group, each substituted with one or more radicals R² and having 5 to 60 aromatic ring atoms; a diarylamino group, diheteroarylamino group or arylheteroarylamino group, each substituted with one or more radicals R² and having 10 to 40 aromatic ring atoms; a combination of the aforementioned systems; or a cross-linkable unit QE which is cross-linked by an acid-catalyzed, base-catalyzed, thermal, or UV cross-linking process in the presence or absence of a photoinitiator or by microwave radiation,
two or more substituents among the substituents R and R' form a mono-or polycyclic, aliphatic, aromatic and/or benzo-condensed ring system with one another,
R², identically or differently in each instance, is
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups are replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³, and
one or more H atoms are replaced with: deuterium, F, Cl, Br, I, CN, CF₃, or NO₂; an aromatic or heteroaromatic ring system, each substituted with one or more radicals R³ and having 5 to 60 aromatic ring atoms; an aryloxy group or heteroaryloxy group, each substituted with one or more radicals R³ and having 5 to 60 aromatic ring atoms; a diarylamino group, diheteroarylamino group or arylheteroarylamino group, each substituted with one or more radicals R³ and having 10 to 40 aromatic ring atoms; or a combination of the aforementioned systems,
two or more substituents R² form a mono- or polycyclic, aliphatic, aromatic and/or benzo-condensed ring system with one another, and
R³, identically or differently in each instance, is H, deuterium, F, CF₃, or an aliphatic, aromatic and/or heteroaromatic hydrocarbon radical having 1 to 20 carbon atoms, wherein one or more H atoms are replaced with F or CF₃, and two or more substituents R³ form a monocyclic or polycyclic aliphatic ring system with one another.

4. The organic molecule of any one of claims 1 to 3, wherein, the organic molecule has
- a value between a lowest excited singlet state (S₁) and a triplet state (T₁) therebelow, △E(S₁-T₁), of less than 3000 cm⁻¹, and/or
- a luminescence lifespan of 100 µs or less.

5. The organic molecule of any one of claims 1 to 4, wherein the organic molecule has a structure of Formula B: 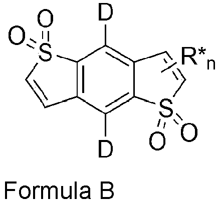 wherein
n is 4, and
outside of this, the definitions set forth in claims 1 to 4 apply.

6. The organic molecule of any one of claims 1 to 4, wherein the organic molecule has a structure of Formula C: 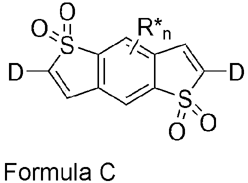 wherein
n is 4, and
outside of this, the definitions set forth in claims 1 to 4 apply.

7. The organic molecule of any one of claims 1 to 4, wherein the organic molecule has a structure of Formula D: 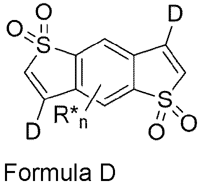 wherein
n is 4, and
outside of this, the definitions set forth in claims 1 to 4 apply.

8. The organic molecule of claim 6, wherein the organic molecule has a structure of Formula E: 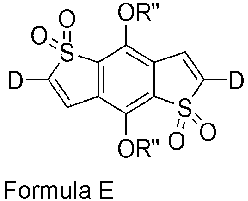 wherein
R" is H, deuterium, CF₃, COOR², CO(NR²)₂, Si(R²)₃, B(OR²)₂, C(=O)R², P(=O)(R²)₂, S(=O)R², S(=O)₂R², O_{S}O₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy or thioalkoxy group having 3 to 40 carbon atoms and being substituted with one or more radicals R²,
one or more non-adjacent CH₂ groups are replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR², and
one or more H atoms are replaced with: deuterium, F, Cl, Br, I, CN, CF₃, or NO₂; an aromatic or heteroaromatic ring system, each substituted with one or more radicals R² and having 5 to 60 aromatic ring atoms; an aryloxy group or heteroaryloxy group, each substituted with one or more radicals R² and having 5 to 60 aromatic ring atoms; a diarylamino group, diheteroarylamino group or arylheteroarylamino group, each substituted with one or more radicals R² and having 10 to 40 aromatic ring atoms; a combination of the aforementioned systems; or a cross-linkable unit QE which is cross-linked by an acid-catalyzed, base-catalyzed, thermal, or UV cross-linking process in the presence or absence of a photoinitiator or by microwave radiation,
two or more substituents among the substituents R and R' form a mono- or polycyclic, aliphatic, aromatic and/or benzo-condensed ring system with one another,
R², identically or differently in each instance, is
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups are replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³, and
one or more H atoms are replaced with: deuterium, F, Cl, Br, I, CN, CF₃, or NO₂; an aromatic or heteroaromatic ring system, each substituted with one or more radicals R³ and having 5 to 60 aromatic ring atoms; an aryloxy group or heteroaryloxy group, each substituted with one or more radicals R³ and having 5 to 60 aromatic ring atoms; or a diarylamino group, diheteroarylamino group or arylheteroarylamino group, each substituted with one or more radicals R³ and having 10 to 40 aromatic ring atoms; or a combination of the aforementioned systems,
two or more substituents R² form a mono- or polycyclic, aliphatic, aromatic and/or benzo-condensed ring system with one another, and
R³, identically or differently in each instance, is H, deuterium, F, CF₃, or an aliphatic, aromatic and/or heteroaromatic hydrocarbon radical having 1 to 20 carbon atoms, wherein one or more H atoms are replaced with F or CF₃, and two or more substituents R³ form a monocyclic or polycyclic aliphatic ring system with one another.

9. The organic molecule of claim 7, wherein the organic molecule has a structure of Formula F: 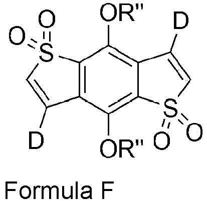 wherein
R" is H, deuterium, CF₃, COOR², CO(NR²)₂, Si(R²)₃, B(OR²)₂, C(=O)R², P(=O)(R²)₂, S(=O)R², S(=O)₂R², O_{S}O₂R², a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy or thioalkoxy group having 3 to 40 carbon atoms and being substituted with one or more radicals R²,
one or more non-adjacent CH₂ groups are replaced with R²C=CR², C=C, Si(R²)₂, Ge(R²)₂, Sn(R²)₂, C=O, C=S, C=Se, C=NR², P(=O)(R²), SO, SO₂, NR², O, S, or CONR², and
one or more H atoms are replaced with: deuterium, F, Cl, Br, I, CN, CF₃, or NO₂; an aromatic or heteroaromatic ring system, each substituted with one or more radicals R² and having 5 to 60 aromatic ring atoms; an aryloxy group or heteroaryloxy group, each substituted with one or more radicals R² and having 5 to 60 aromatic ring atoms; a diarylamino group, diheteroarylamino group or arylheteroarylamino group, each substituted with one or more radicals R² and having 10 to 40 aromatic ring atoms; a combination of the aforementioned systems; or a cross-linkable unit QE which is cross-linked by an acid-catalyzed, base-catalyzed, thermal, or UV cross-linking process in the presence or absence of a photoinitiator or by microwave radiation,
two or more substituents among the substituents R and R' form a mono- or polycyclic, aliphatic, aromatic and/or benzo-condensed ring system with one another,
R², identically or differently in each instance, is
H, deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, a linear alkyl group, alkoxy group or thioalkoxy group having 1 to 40 carbon atoms, a linear alkenyl group or alkynyl group having 2 to 40 carbon atoms, or a branched or cyclic alkyl group, alkenyl group, alkynyl group, alkoxy group or thioalkoxy group having 3 to 40 carbon atoms and being substituted with one or more radicals R³,
one or more non-adjacent CH₂ groups are replaced with R³C=CR³, C=C, Si(R³)₂, Ge(R³)₂, Sn(R³)₂, C=O, C=S, C=Se, C=NR³, P(=O)(R³), SO, SO₂, NR³, O, S, or CONR³, and
one or more H atoms are replaced with: deuterium, F, Cl, Br, I, CN, CF₃, or NO₂; an aromatic or heteroaromatic ring system, each substituted with one or more radicals R³ and having 5 to 60 aromatic ring atoms; an aryloxy group or heteroaryloxy group, each substituted with one or more radicals R³ and having 5 to 60 aromatic ring atoms; a diarylamino group, diheteroarylamino group or arylheteroarylamino group, each substituted with one or more radicals R³ and having 10 to 40 aromatic ring atoms; or a combination of the aforementioned systems,
two or more substituents R² form a mono- or polycyclic, aliphatic, aromatic and/or benzo-condensed ring system with one another, and
R³ is the same or differently in each instance H, deuterium, F, CF₃, or an aliphatic, aromatic and/or heteroaromatic hydrocarbon radical having 1 to 20 carbon atoms, wherein one or more H atoms are replaced with F or CF₃, and two or more substituents R³ form a monocyclic or polycyclic aliphatic ring system with one another.

10. The organic molecule of claim 1 or any one of claims 3 to 9, wherein D is selected from the group consisting of substituted and unsubstituted carbazole, substituted and unsubstituted indole, substituted and unsubstituted indoline, substituted and unsubstituted 2,3,4,9-tetrahydrocarbazole, substituted and unsubstituted 1,2,3,4-tetrahydroquinoline, substituted and unsubstituted phenothiazine, substituted and unsubstituted phenothiazine, substituted and unsubstituted dihydrophenazine, and substituted and unsubstituted spiro-compounds.

11. The organic molecule of claim 1 or any one of claims 3 to 10, wherein the electron donor group of Formula IV has a structure of Formula V: 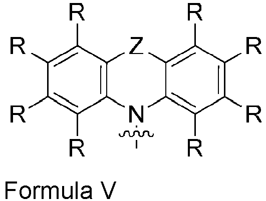 wherein R is as defined in claim 1.

12. The organic molecule of claim 11, wherein Z is a direct bond.

13. A method of preparing the organic molecule of any one of claims 1 to 12, the method comprising catalytically coupling a bromine-substituted compound with a secondary amine or amine-substituted aromatic, particularly by Cu-, Ni-, or Pd-catalyzed coupling, wherein, in the latter coupling, nickel-catalyzed coupling is performed according to one of the following reaction schemes: 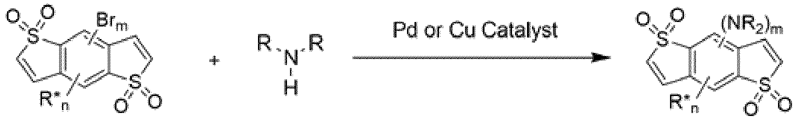 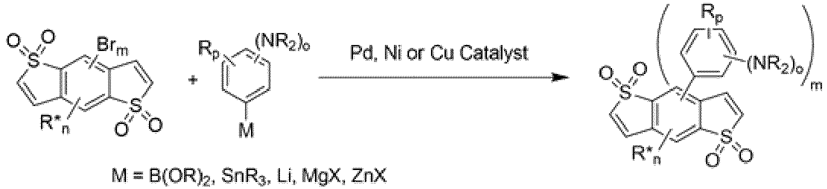 wherein the definitions set forth in claim 1 apply, and X is Cl, Br, or I.

14. The method of claim 12, further comprising substituting the donor group D, radical R" and/or radical R with at least one cross-linkable unit by an acid-catalyzed, base-catalyzed, thermal, or UV cross-linking process, wherein the cross-linkable unit is cross-linked in the presence or absence of a photoinitiator or by microwave radiation, and is selected from the following group:
oxetane, azide, alkyne, and alkene derivatives for click reactions:

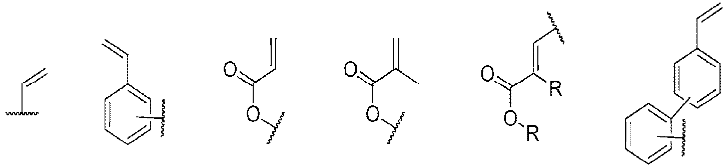

15. Use of the organic molecule of any one of claims 1 to 12, as an emitter or absorber in an optoelectronic device prepared by a vacuum evaporation process or from a solution.

16. An optoelectronic device comprising the organic molecule of claims 1 to 12.

17. A method of preparing an optoelectronic device comprising the organic molecule of claims 1 to 12.
